# EUROPÄISCHE PATENTSCHRIFT

(11) **EP 1 653 933 B1**
(45) Veröffentlichungstag und Bekanntmachung des Hinweises auf die Patenterteilung: **08.11.2006**
(21) Anmeldenummer: 04763415.9
(22) Anmeldetag: 23.07.2004
(51) Int. Cl.: A61K 9/70, A61K 31/00, A61K 47/24, A61L 15/16

(54) **DERMALES ODER TRANSDERMALES THERAPEUTISCHES SYSTEM ENTHALTEND EIN ORMOCER MIT BARRIEREWIRKUNG AUF EINE ABDECKFOLIE**
DERMAL OR TRANSDERMAL THERAPEUTIC SYSTEM COMPRISING AN ORMOCER WITH BARRIER EFFECT ON A COVER FOIL
SYSTEME THERAPEUTIQUE DERMIQUE OU INTRADERMIQUE COMPORTANT UN ORMOCER A EFFET BARRIERE SUR UN FILM DE RECOUVREMENT

(30) Priorität: 07.08.2003 DE 10336211; 11.06.2004 DE 102004028415
(43) Veröffentlichungstag der Anmeldung: 10.05.2006
(73) Patentinhaber: LTS LOHMANN Therapie-Systeme AG, 56626 Andernach (DE)
(72) Erfinder: THEOBALD, Frank, 53498 Bad Breisig (DE); WEBER, Notger, 56567 Neuwied (DE); SIMON, Günter, 54576 Hillesheim (DE); AMBERG-SCHWAB, Sabine, 97250 Erlabrunn (DE); WEBER, Ulrike, 97297 Waldbüttelbrunn (DE)
(74) Vertreter: Schmidt, Werner
(86) Internationale Anmeldenummer: PCT/EP2004/008221
(87) Internationale Veröffentlichungsnummer: WO 2005/016320

(56) Entgegenhaltungen:
- WO-A-02/34510
- DE-A- 19 958 554
- AMBERG-SCHWAB S ET AL: "Inorganic-organic polymers as migration barriers against liquid and volatile compounds" J SOL GEL SCI TECHNOL; JOURNAL OF SOL-GEL SCIENCE AND TECHNOLOGY JANUARY/MARCH 2003, Bd. 26, Nr. 1-3, Januar 2003 (2003-01), Seiten 699-703, XP001204606
- KNITTEL D ET AL: "Surface of textiles and the human skin: 1. Surface modification of fibers as therapeutic and diagnostic systems" EXOGENOUS DERMATOLOGY 2003 SWITZERLAND, Bd. 2, Nr. 1, 2003, Seiten 11-16, XP009041824 ISSN: 1424-4616

## Beschreibung

Die vorliegende Erfindung betrifft ein dermales oder transdermales therapeutisches System enthaltend eine Abdeckfolie mit Barrierewirkung gegen Gase, Aromen und leicht flüchtige Stoffe, das dadurch gekennzeichnet ist, dass die Abdeckfolie aus mindestens einer Trägerschicht und einer Schicht aus einem anorganisch-organischen Hybridpolymeren (Ormocer^{®}) besteht.

Dermale therapeutische Systeme sind solche, die einen pharmazeutischen, topisch wirkenden Stoff in Kontakt mit dem erkrankten Hautabschnitt bzw. dem darunter liegenden erkrankten Muskelringen bringen; dazu gehören alle sogenannten medizinischen Pflaster. Transdermale therapeutische Systeme hingegen befördern den Wirkstoff durch die Haut hindurch in den Blutkreislauf und wirken somit systemisch.
Beide Systeme bestehen im wesentlichen aus einer Abdeckfolie (Rückschicht, Backinglayer), einem Wirkstoffreservoir bzw. einer wirkstoffhaltigen Matrix und einer abziehbaren Schutzfolie (Release liner). Die Abdeckfolie bleibt auch während der Applikation am dermalen bzw. transdermalen therapeutischen System, um die wirkstoffhaltige Matrix oder das wirkstoffhaltige Reservoir einerseits mechanisch zu schützen, andererseits den Wirkstoff entweder vor dem Einfluss von Gasen z. B. Sauerstoff und Wasserdampf zu schützen oder das teilweise Verdampfen eines leicht flüchtigen Wirkstoffs zu verhindern.

Sowohl die Abdeckfolie, die als Rückschicht dient, als auch die abziehbare Schutzfolie bestehen im allgemeinen aus organischen Polymeren. Geeignete Materialien dafür sind beispielsweise: Polyethylen niederer Dichte (LDPE) und hoher Dichte (HDPE), Polypropylen (PP), Polyamid (PA), Polyvinylchlorid (PVC), Polyvinylester, Polyethylentherephtalat (PET), Copolymere aus mindestens zwei dieser Polymere oder Mischungen dieser Polymere. Da die Abdeckfolie während der Anwendung auf der Haut verbleibt, sollte sie möglichst flexibel und elastisch sein, um einen entsprechenden Tragekomfort bei Patienten zu gewährleisten.

Starre Folien führen zur Faltenbildung und zum Einschneiden in die Haut. Als flexible und elastische Abdeckfolien kommen insbesondere LDPE und HDPE infrage. Alle vorstehend genannten Polymere, insbesondere aber die beiden Polyethylene besitzen jedoch den Nachteil, dass sie mehr oder weniger gute Speichersysteme für Wirkstoffe darstellen. Dadurch besteht die Gefahr, dass Wirkstoff aus dem Reservoir oder der wirkstoffhaltigen Matrix während der Lagerung und/oder der Anwendung über den Gasraum in die Abdeckfolie bis zur Sättigungskonzentration migriert. Dadurch wird der für die Permeation durch die Haut in der Matrix bzw. dem Reservoir zur Verfügung stehende Wirkstoff minimiert und die Abgabekinetik des dermalen bzw. transdermalen therapeutischen Systems minimiert.

In DE 199 22 368A1 werden Polymerzusammensetzungen aus wasserlöslichen Polymeren und gebundenen Siliciumdioxid-Bausteirien, die durch Polymerisation verbunden sind, als Material für pharmazeutische Trägermaterialien beschrieben. Diese Zusammensetzungen entsprechen jedoch nicht derjenigen der Ormocer^{®}e.

In DE 195 19 593 wird ein transdermales therapeutisches System für die Abgabe von flüchtigen Wirkstoffen beschrieben, worin eine für den Wirkstoff im wesentlichen undurchlässigen Rückschicht, welche aus einem thermoplastischen organischen Polymer besteht, enthalten ist.

WO 95/07817 beschreibt einen für Sauerstoff und Feuchtigkeit undurchlässigen Mehrschichtfilm, hergestellt durch Extraktion von Nylon und/oder Copolymeren aus Ethylen und Vinylalkohol zusammen mit einem thermoplastischen organischen Polymer.

DE 199 58 554 A offenbart ein Transdermales Therapeutisches System, umfassend eine für Wirkstoffe undurchlässige Rückschicht, mindestens eine Polymerschicht mit darin enthaltenden Mikroreservoiren und mindestens einen Wirkstoff und eine vor Gebrauch zu entfernende Schutzschicht. Das Material der Rückschicht besteht aus Folien wie z.B. Polyethylen, Polypropylen, Polyester oder aus Laminaten unterschiedlicher Polymere.

Amberg-Schwab S. et al: "Inorganic-organic polymers as migration barrieres against liquid and volatile compounds", Journal of Sol-Gel Science and Technologie 26, 699-703, 2003. Die Veröffentlichung beschreibt die Untersuchung von verschiedenen, photochemisch härtbaren Hybridpolymeren, synthetisiert auf Basis der Sol-Gel-Technik, als neues Beschichtungsmaterial zum Einsatz als Migrationsbarrieren mit guten Eigenschaften zu Bedruckbarkeit, Abriebsfestigkeit und Antistatik für die Verpackungsindustrie.

WO 02/34510 A1 offenbart ein flexibles Sterilgut-Verpackungsmaterial aus einer Folie oder einem Folienverbund mit Barriereeigenschaften gegenüber Gasen, Wasserdampf und Aromastoffen enthaltend eine Trägerfolie und darauf angeordnet eine keramische Dünnschicht, auf der wiederum eine funktionelle Schicht aus einem anorganisch-organischen Hybridpolymer aufgetragen ist. Das Material eignet sich für die Sterilisation bei hohen Temperaturen.

Knittel D. et al: "Surface of Textiles and the Human Skin: 1. Surface Modification of Fibers as Therapeutic and Diagnostic Systems", Exogenous Dermatology 2003; 2, 11-16. Die Veröffentlichung beschreibt eine neue Methode zur Veredelung hautseitiger Oberflächen von Textilen mit Cyclodextrinen durch Verankerung auf der Textiloberfläche. Weiter wird vorgeschlagen, die Textilien mit wirkstoffhaltigen, anorganisch-organischen Hybridsystemen zu funktionalisieren, um als Depot für eine kontrollierte Freisetzung zu dienen.

Es ist ferner bereits vorgeschlagen worden, die Durchlässigkeit von flexiblen bzw. elastischen Polymerschichten für Gase oder flüchtige Wirkstoffe durch die Kombination von Polymeren mit Metallschichten, z. B. durch eine Aluminiumbeschichtung, oder auch durch Beschichtung mit Metalloxiden herabzusetzen oder ganz zu unterbinden. Leider wird durch eine solche Kombination jedoch die Flexibilität, insbesondere aber die Elastizität solcher Systeme stark beeinträchtigt.

Die vorliegende Erfindung hat sich daher die Aufgabe gestellt, dermale oder transdermale therapeutische Systeme mit Abdeckfolien, welche eine gute Flexibilität und gleichzeitig eine hervorragende Barrierewirkung hinsichtlich leicht flüchtiger Wirkstoffe bzw. Wirkstoffen mit hohem Dampfdruck besitzen, zu entwickeln.

Diese Aufgabe wird erfindungsgemäß dadurch gelöst, dass man als Abdeckfolien solche aus organischen Polymeren verwendet, die mit einem anorganisch-organischen Hybridpolymeren, einem sogenannten Ormocer^{®}, beschichtet sind.

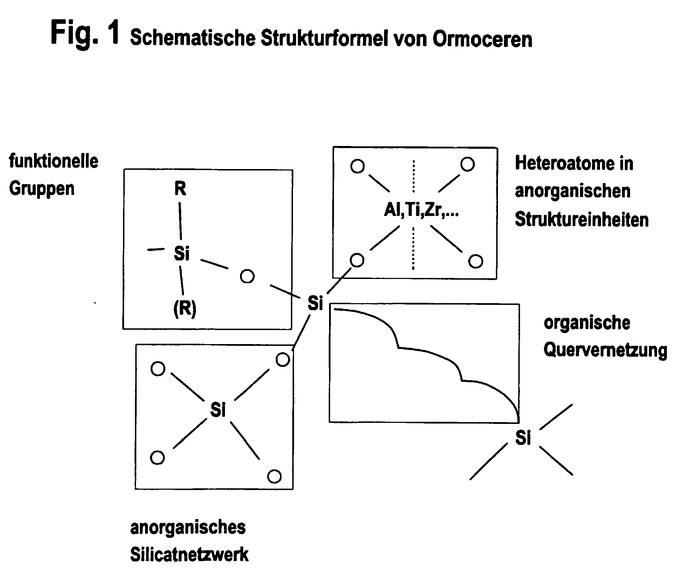

Anorganisch-organische Hybridpolymere, sogenannte Ormocer^{®}e, sind seit einigen Jahren bekannt. Sie werden in zwei Stufen auf die folgende Weise hergestellt: Zunächst Aufbau eines anorganischen Netzwerkes durch kontrollierte Hydrolyse und Kondensation von organisch modifizierten Siliciumoxiden, wobei auch eine Cokondensation mit anderen Metallalkoxiden (Ti-, Zr-, Al-Alkoxiden) möglich ist. Dann, in einem zweiten Schritt, reagieren die polymerisierbaren Gruppen, die mit dem anorganischen Netzwerk verbunden sind, miteinander in der Folge von thermischer oder UV-Behandlung. Ein solches Hybridpolymer besitzt dann die in der Figur 1 wiedergegebenen schematischen Strukturformel.

Herstellung und Eigenschaften von Ormocer^{®}en sind in den im folgenden aufgeführten Veröffentlichungen beschrieben worden:
EP 0 358 011 A2; EP 0 373 451 A1; EP 0 610 831 A2; EP 0 644 908 B1; EP 0 792 846 A1; EP 0 934 989 A
Diese Veröffentlichungen werden hier ausdrücklich als Teil der Offenbarung genannt.

Während es bisher nicht gelungen ist, Abdeckfolien für dermale oder transdermale Systeme mit guten Barriereeigenschaften und gleichzeitig befriedigenden elastischen Eigenschaften herzustellen, wurde nun überraschenderweise gefunden, dass eine Beschichtung von Polymerfolien mit Ormocer^{®}en, deren gute Barrierewirkung bereits bekannt ist, zu Abdeckfolien mit einer nicht oder nur unwesentlich beeinträchtigten Elastizität führen. Besonders geeignet sind Ormocer^{®}-Schichten, die eine Dicke zwischen 1 µm und 10 µm aufweisen.

Sowohl die Abdeckfolie, die als Rückschicht dient, als auch die abziehbare Schutzfolie bestehen im allgemeinen vorzugsweise aus organischen Polymeren. Geeignete Materialien dafür sind beispielsweise: Polyethylen niederer Dichte (LDPE) und hoher Dichte (HDPE), Polypropylen (PP), Polyamid (PA), Polyvinylchlorid (PVC), Polyvinylester, Polyester, Polyethylentherephtalat (PET), Copolymere aus mindestens zwei dieser Polymere oder Mischungen dieser Polymere. Da die Abdeckfolie während der Anwendung auf der Haut verbleibt, sollte sie möglichst flexibel und elastisch sein, um einen entsprechenden Tragekomfort bei Patienten zu gewährleisten. Starre Folien führen zur Faltenbildung und zum Einschneiden in die Haut. Als flexible und elastische Abdeckfolien kommen insbesondere LDPE und HDPE infrage.

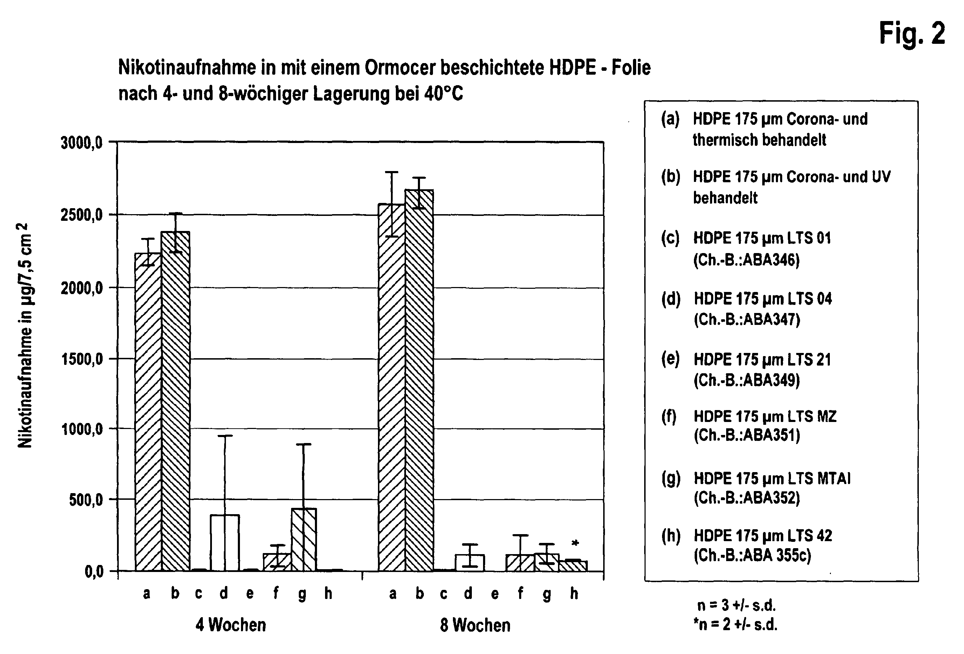

Zur Untersuchung der Eignung von mit einem Ormocer^{®}-beschichteten flexiblen Abdeckfolien wurde eine 175 µm Dicke HDPE-Folie mit einem Ormocer^{®}-Lack der oben genannten Zusammensetzung beschichtet. Die beidseitig beschichteten Folien wurden in einen Gasraum mit Nikotin eingebracht und die Wirkstoffaufnahme der Folien nach 4- und 8-wöchiger Inkubationszeit bei 40°C bestimmt. Die Ergebnisse der Untersuchungen sind in der Figur 2 dargestellt.

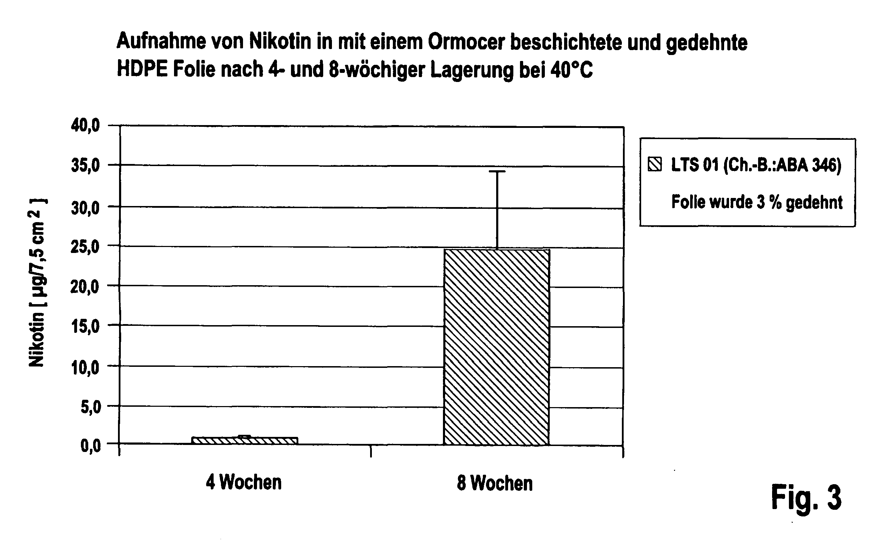

Ebenso konnte gezeigt werden, dass die erfindungsgemäßen Abdeckfolien eine gute Dehnbarkeit, das heißt also Elastizität besitzen und die Barriereeigenschaften auch im gedehnten Zustand erhalten bleiben. Um das zu zeigen, wurde eine mit dem Hybridpolymer Ormocer^{®} beschichtete HDPE-Folie um 3 % gedehnt und die Wirkstoffaufnahme nach 4- und 8-wöchiger Exposition gegenüber Nikotin bestimmt. Die Ergebnisse sind in Figur 3 dargestellt.

## Patentansprüche

1. Dermales oder transdermales therapeutisches System umfassend eine abziehbare Schutzschicht, eine mindestens einen Wirkstoff enthaltende Reservoir- oder Matrixschicht und eine Abdeckfolie mit Barrierewirkung gegen Gase, Aromen und leicht flüchtige Stoffe, **dadurch gekennzeichnet, dass** die Abdeckfolie aus mindestens einer Trägerschicht und mindestens einer Schicht bestehend aus einem Ormocer besteht.

2. Dermales oder transdermales therapeutisches System gemäß Anspruch 1, **dadurch gekennzeichnet, dass** das Ormocer erhalten wird durch hydrolytische Kondensation von organisch modifizierten Siliciumdioxiden.

3. Dermales oder transdermales therapeutisches System gemäß Anspruch 1 und 2, **dadurch gekennzeichnet, dass** die Trägerschicht aus mindestens einem organischen Polymer besteht.

4. Dermales oder transdermales therapeutisches System gemäß Anspruch 3, **dadurch gekennzeichnet, dass** das organische Polymer aus Polyethylen, niederer oder hoher Dichte, aus Polypropylen, Polyamid, Polyvinylchlorid, Polyvinylester, Polyester, einer Mischung aus mindestens zwei dieser Polymere oder mindestens einem Copolymeren aus mindestens zwei Monomeren der vorstehend genannten Polymere besteht.

5. Dermales oder transdermales therapeutisches System gemäß mindestens einem der vorhergehenden Ansprüche, **dadurch gekennzeichnet, dass** die Trägerschicht aus Polyethylentherephtalat besteht.

6. Dermales oder transdermales therapeutisches System gemäß mindestens einem vorhergehenden Ansprüche, **dadurch gekennzeichnet, dass** die Ormocer-Schicht eine Dicke zwischen 1 µm und 10 µm aufweist.

7. Dermales oder transdermales therapeutisches System gemäß mindestens einem der vorhergehenden Ansprüche, **dadurch gekennzeichnet, dass** die Ormocer-Schicht so auf der Trägerschicht aufgebracht ist, dass sie an die wirkstoffhaltige Schicht angrenzt.

8. Dermales oder transdermales therapeutisches System gemäß mindestens einem der vorhergehenden Ansprüche, **dadurch gekennzeichnet, dass** auf beiden Oberflächen der Trägerschicht jeweils mindestens eine Ormocerschicht aufgebracht ist.

9. Dermales oder transdermales therapeutisches System gemäß mindestens einem der vorhergehenden Ansprüche, **dadurch gekennzeichnet, dass** es einen leichtflüchtigen Wirkstoff enthält.

10. Dermales oder transdermales therapeutisches System gemäß Anspruch 9, **dadurch gekennzeichnet, dass** der Wirkstoff Nikotin ist.

## Claims

1. A dermal or transdermal therapeutic system comprising a peelable protective layer, a reservoir or matrix layer containing at least one active compound and a covering film having a barrier effect against gases, aromas and readily volatile substances, **characterized in that** the covering film is composed of at least one supporting layer and at least one layer composed of an ormocer.

2. The dermal or transdermal therapeutic system as claimed in claim 1, **characterized in that** the ormocer is obtained by the hydrolytic condensation of organically modified silicon dioxides.

3. The dermal or transdermal therapeutic system as claimed in claims 1 and 2, **characterized in that** the supporting layer is composed of at least one organic polymer.

4. The dermal or transdermal therapeutic system as claimed in claim 3, **characterized in that** the organic polymer is composed of low density or high density polyethylene, or of polypropylene, polyamide, polyvinyl chloride, polyvinyl ester or polyester, of a mixture of at least two of these polymers or of at least one copolymer composed of at least two monomers of the abovementioned polymers.

5. The dermal or transdermal therapeutic system as claimed in at least one of the preceding claims, **characterized in that** the supporting layer is composed of polyethylene terephthalate.

6. The dermal or transdermal therapeutic system as claimed in at least one of the preceding claims, **characterized in that** the ormocer layer has a thickness of between 1 µm and 10 µm.

7. The dermal or transdermal therapeutic system as claimed in at least one of the preceding claims, **characterized in that** the ormocer layer is applied on the supporting layer such that it is adjacent to the active compound-containing layer.

8. The dermal or transdermal therapeutic system as claimed in at least one of the preceding claims, **characterized in that** in each case at least one ormocer layer is applied on both surfaces of the supporting layer.

9. The dermal or transdermal therapeutic system as claimed in at least one of the preceding claims, **characterized in that** it comprises a readily volatile active compound.

10. The dermal or transdermal therapeutic system as claimed in claim 9, **characterized in that** the active compound is nicotine.

## Revendications

1. Système thérapeutique dermique ou transdermique comprenant une couche protectrice amovible, au moins une couche réservoir ou matricielle renfermant le principe actif et une feuille de recouvrement avec effet barrière aux gaz, aux arômes et aux substances très volatiles, **caractérisé en ce que** la feuille de recouvrement est constituée par une couche support et par au moins une couche en un ormocer.

2. Système thérapeutique dermique ou transdermique selon la revendication 1, **caractérisé en ce que** l'ormocer est obtenu par condensation hydrolytique de dioxydes de silicium organiquement modifiés.

3. Système thérapeutique dermique ou transdermique selon la revendication 1 et 2, **caractérisé en ce que** la couche support est constituée par au moins un polymère organique.

4. Système thérapeutique dermique ou transdermique selon la revendication 3, **caractérisé en ce que** le polymère organique est constitué par un polyéthylène basse densité ou haute densité, un polypropylène, un polyamide, un poly(chlorure de vinyle), un poly(ester de vinyle), un polyester, un mélange d'au moins deux de ces polymères ou par au moins un copolymère d'au moins deux monomères des polymères précités.

5. Système thérapeutique dermique ou transdermique selon au moins l'une des revendications précédentes, **caractérisé en ce que** la couche support est en poly(téréphtalate d'éthylène).

6. Système thérapeutique dermique ou transdermique selon au moins l'une des revendications précédentes, **caractérisé en ce que** la couche d'ormocer présente une épaisseur comprise entre 1 µm et 10 µm.

7. Système thérapeutique dermique ou transdermique selon au moins l'une des revendications précédentes, **caractérisé en ce que** la couche d'ormocer est appliquée à la couche substrat de façon telle qu'elle soit limitrophe à la couche renfermant le principe actif.

8. Système thérapeutique dermique ou transdermique selon au moins l'une des revendications précédentes, **caractérisé en ce qu'**on applique au moins une couche d'ormocer aux deux surfaces de la couche substrat.

9. Système thérapeutique dermique ou transdermique selon au moins l'une des revendications précédentes, **caractérisé en ce qu'**il renferme un principe actif très volatil.

10. Système thérapeutique dermique ou transdermique selon la revendication 9, **caractérisé en ce que** le principe actif est la nicotine.
